# EUROPÄISCHE PATENTSCHRIFT

(11) **EP 1 527 057 B1**
(45) Veröffentlichungstag und Bekanntmachung des Hinweises auf die Patenterteilung: **21.11.2007**
(21) Anmeldenummer: 03765087.6
(22) Anmeldetag: 22.07.2003
(51) Int. Cl.: C07D 301/12, C07C 29/10, C07C 31/20, B01D 3/40

(54) **VERFAHREN ZUR KONTINUIERLICHEN HERSTELLUNG VON PROPYLENGLYKOLEN**
METHOD FOR THE CONTINUOUS PRODUCTION OF PROPYLENE GLYCOL
PROCEDE DE FABRICATION CONTINUE DE PROPYLENE GLYCOLS

(30) Priorität: 23.07.2002 DE 10233385
(43) Veröffentlichungstag der Anmeldung: 04.05.2005
(73) Patentinhaber: BASF Aktiengesellschaft, 67056 Ludwigshafen (DE)
(72) Erfinder: BASSLER, Peter, 68519 Viernheim (DE); GÖBBEL, Hans-Geeorg, 67169 Kallstadt (DE); TELES, Joaquim, Henrique, 67166 Otterstadt (DE); RUDOLF, Peter, 68526 Ladenburg (DE)
(74) Vertreter: Hörschler, Wolfram Johannes
(86) Internationale Anmeldenummer: PCT/EP2003/007988
(87) Internationale Veröffentlichungsnummer: WO 2004/009568

(56) Entgegenhaltungen:
- EP-A- 0 031 537
- EP-A- 0 780 147
- WO-A-00/07965
- DE-A- 10 022 465
- GB-A- 2 068 408
- US-A- 4 937 393

## Beschreibung

Die Erfindung betrifft ein Verfahren zur kontinuierlichen Herstellung von Propylenglykolen durch Umsetzung von Propylen mit Wasserstoffperoxid zu Propylenoxid unter gleichzeitiger oder nachgeschalteter Glykolbildung unter gemeinsamer Aufarbeitung. Die Abtrennung der Glykole aus dem Produktgemisch erfolgt destillativ, wobei in einer Trennwandkolonne mit zwei Seitenabzügen Propylenglykol und Dipropylenglykol und in einer mit der Trennwandkolonne thermisch gekoppelten Kolonne Tripropylenglykol abgetrennt werden. Die Erfindung betrifft auch eine Vorrichtung zur Durchführung des Verfahrens.

Propylenglykol und Polypropylenglykole sind wichtige Zwischenprodukte beispielsweise zur Herstellung von Polyester- und Epoxidharzen sowie PolyurethanSchaumstoffen. Für ihre technische Herstellung ist bisher ein dreistufiges Verfahren bedeutsam.

Hierbei wird in einer ersten Stufe Propylenoxid mit einem stöchiometrischen Überschuss an Wasser bei einer Temperatur von 180 bis 220°C und einem Druck zwischen 15 und 25 bar umgesetzt. Für diese Umsetzung werden im Allgemeinen hintereinander geschaltete Rohrreaktoren eingesetzt. Die entstehenden Propylenglykole liegen dann in Form eines Gemisches vor, in dem Propylenglykol, das auch als Monopropylenglykol oder 1,2-Propylenglykol bezeichnet wird, Di- und Tripropylenglykol etwa im Gewichtsverhältnis 100:10:1 enthalten sind. Dieses Gemisch wird in einer zweiten Stufe in eine Entwässerungsapparatur überführt, in der in einer mehrstufigen Verdampfung das überschüssige Wasser abgetrennt und in die Reaktion zurückgeführt wird. Schließlich erfolgt in einer dritten Stufe die Reindestillation der Propylenglykole. Dabei werden gemäß dem Stand der Technik drei in Reihe geschaltete Kolonnen mit jeweils einem Seitenabzug eingesetzt.

In der ersten Kolonne wird aus dem Seitenabzug das Propylenglykol erhalten, wobei der Sumpf in die zweite Kolonne überführt wird, aus deren Seitenabzug das Dipropylenglykol erhalten wird. Das Sumpfgemisch dieser zweiten Kolonne wird in die dritte Kolonne überführt, aus deren Seitenabzug schließlich das Tripropylenglykol erhalten werden kann (Ullmann's Encyclopädie der technischen Chemie, 4. Auflage, Band 19, Seite 425-432).

Das beschriebene Verfahren erfordert insbesondere im Verfahrensschritt der destillativen Abtrennung der drei genannten Propylenglykole einen hohen apparativen und energetischen Aufwand, da drei Kolonnen verwendet werden. Ferner setzt es die Verwendung reinen Propylenoxids als Ausgangsprodukt voraus.

US 4 937 393 betrifft ein Verfahren zur Herstellung von Propylenglykol aus reinem Propylenoxid und Wasser.

Es war die Aufgabe gestellt, ein verbessertes Verfahren zur Herstellung der Propylenglykole bereitzustellen. Insbesondere sollte sich beim erfindungsgemäßen Verfahren als Edukt auch Propylenoxid minderer Reinheit einsetzen sowie die Abtrennung der genannten Propylenglykole mit vermindertem apparativem und energetischem Aufwand durchführen lassen.

Diese Aufgabe konnte mit einem Verfahren gelöst werden, bei dem der Herstellungsprozess für Propylenoxid, bei dem bereits anteilig Propylenglykole als Nebenprodukte gebildet werden, sowie die Umsetzung des Propylenoxids mit Wasser gekoppelt werden. Vorzugsweise wird dabei das Propylenoxid durch Umsetzung von Propylen mit Peroxiden, beispielsweise Wasserstoffperoxid, hergestellt. Die Abtrennung der Propylenglykole erfolgt durch Destillation in einer Trennwandkolonne mit zwei flüssigen Seitenabzügen und einer damit thermisch gekoppelten Kolonne.

Gegenstand der Erfindung ist somit ein Verfahren zur kontinuierlichen Herstellung von Propylenglykolen, dadurch gekennzeichnet, dass es mindestens die Stufen (i) bis (iii) umfasst:
(i) Umsetzung von Propylen mit Wasserstoffperoxid zu Propylenoxid und Propylenglykolen,
(ii) Umsetzung des in Stufe (i) erhaltenen Propylenoxids mit Wasser zu Propylenglykolen,
(iii) Abtrennung der in den Stufen (i) und (ii) erhaltenen Propylenglykole.

In einer bevorzugten Ausführung ist das erfindungsgemäße Verfahren dadurch gekennzeichnet, dass in Stufe (iii) die Abtrennung destillativ in einer Trennwandkolonne mit zwei Seitenabzügen und einer damit thermisch gekoppelten Kolonne erfolgt, wobei aus dem oberen Seitenabzug der Trennwandkolonne Propylenglykol, aus dem unteren Seitenabzug Dipropylenglykol und aus der damit thermisch gekoppelten Kolonne Tripropylenglykol erhalten wird.

Nach dem neuen Verfahren ist es somit nicht mehr erforderlich, reines Propylenoxid in die Umsetzung mit Wasser einzusetzen, sondern es kann das bei der Synthese von Propylenoxid aus Propylen und Wasserstoffperoxid anfallende Rohprodukt, das bereits anteilig die Propylenglykole enthält, verwendet werden. Durch Verwendung der Trennwandkolonne in Kombination mit der thermisch gekoppelten Kolonne kann zusätzlich im Vergleich zum beim Stand der Technik beschriebenen Verfahren eine erhebliche Einsparung des apparativen und energetischen Aufwandes erreicht werden, da anstatt drei nur noch zwei Kolonnen für die Abtrennung der Glykole notwendig sind. Das kontinuierlich durchgeführte Verfahren bietet somit im Vergleich zum Verfahren des Standes der Technik Vorteile.

Die Herstellung von Propylenoxid durch Umsetzung von Propylen mit Wasserstoffperoxid in Stufe (i) ist bekannt und kann nach bekannten Methoden durchgeführt werden. Dabei können sowohl einstufige wie auch mehrstufige Verfahren zum Einsatz kommen. Wegen der Wirtschaftlichkeit wird mehrstufigen Verfahren der Vorzug gegeben.

Beispielsweise sieht das in der WO 00/07965 beschriebene mehrstufige Verfahren vor, dass die Umsetzung von Propylen mit Wasserstoffperoxid zu Propylenoxid mindestens die Stufen (α) bis (γ) umfasst:
(α) Umsetzung von Propylen mit Wasserstoffperoxid unter Erhalt einer Mischung, umfassend Propylenoxid und nicht umgesetztes Wasserstoffperoxid,
(β) Abtrennung des nicht umgesetzten Wasserstoffperoxids aus der aus Stufe (α) resultierenden Mischung,
(γ) Umsetzung des abgetrennten Wasserstoffperoxids aus Stufe (β) mit Propylen.

Demgemäss findet in dieser Reaktionssequenz die Umsetzung von Propylen mit Wasserstoffperoxid in mindestens zwei Stufen (α) und (γ) statt, wobei das in Stufe (β) abgetrennte Wasserstoffperoxid erneut in die Reaktion eingesetzt wird.

Vorzugsweise wird in dieser Sequenz besagte Umsetzung an einem heterogenen Katalysator durchgeführt, wobei auch ein Lösungsmittel, wie beispielsweise Methanol, mitverwendet werden kann.

Vorzugsweise erfolgen dabei die Umsetzungen in den Stufen (α) und (γ) in zwei getrennten Reaktoren. Zumeist werden hierzu Festbettreaktoren verwendet, wobei die Umsetzung der Stufe (α) in einem isothermen und die Umsetzung der Stufe (γ) in einem adiabatischen Reaktor erfolgt.

Der Wasserstoffperoxid-Umsatz in Stufe (α) erreicht im Allgemeinen ca. 85 % bis 90 % und in Stufe (γ), ca. 95 % bezogen auf Stufe (β). In der Summe kann über beide Stufen ein Wasserstoffperoxidumsatz von ca. 99 % bei einer Propylenoxid-Selektivität von ca. 94-95 % erreicht werden.

Das bei der Umsetzung erhaltene Gemisch weist im Allgemeinen einen Gehalt von ca. 6 bis 10 Gew.-% an Propylenoxid auf. Es enthält als weitere Komponenten beispielsweise Methanol als Lösungsmittel, Wasser, nichtumgesetztes Propylen als organische Verbindung und Wasserstoffperoxid sowie Acetaldehyd und Methylformiat.

Das im Produktgemisch enthaltene Wasser stammt aus der Umsetzungs- und Zersetzungsreaktion des Wasserstoffperoxids. Außerdem wird in den Stufen (α) und (γ) vorzugsweise eine wässerige Wasserstoffperoxidlösung eingesetzt. Unter den vorliegenden Bedingungen vermag Wasser mit Propylenoxid zu Propylenglykol zu reagieren. Das somit als Nebenprodukt gebildete Glykol kann nun seinerseits mit weiterem Propylenoxid zu den Di- und Tripropylenglykolen weiterreagieren. Damit liegen neben dem Propylenoxid auch bereits Mono-, Di- und Tripropylenglykol vor. Diese Glykole können aus dem das Propylenoxid enthaltenden Produktgemisch gut destillativ abgetrennt werden, da sie relativ hoch sieden. Bei Destillation in einer Kolonne werden sie mit dem Sumpf erhalten, während über den Kopf der Kolonne Propylenoxid zusammen mit weiteren flüchtigen Anteilen abdestilliert wird. Dieses Propylenoxid ist etwa 95 bis 99 %-ig und wird als Rohpropylenoxid bezeichnet.

Mit dem Sumpf werden auch die durch Folgereaktion von Propylenoxid mit Methanol unter den Reaktionsbedingungen als weitere Nebenprodukte entstandenen 1- und 2-Methoxypropanole erhalten. Als weitere Nebenprodukte sind im Sumpfaustrag auch 2-Hydroperoxy-1-propanol und 1-Hydroperoxy-2-propanol enthalten, welche durch Addition von Wasserstoffperoxid an Propylenoxid entstehen.

Es ist bevorzugt, diese Hydroperoxyalkohole zu reduzieren, wobei gleichfalls Propylenglykol gebildet wird. Beispielsweise können zur Reduktion die in der DE 101 05 527.7 beschriebenen Methoden verwendet werden. Vorzugsweise kommt als Reduktionsmethode die katalytische Hydrierung in Betracht.

Somit ist das erfindungsgemäße Verfahren auch dadurch gekennzeichnet, dass in Stufe (i) als Nebenprodukt Propylenglykol durch Reduktion von 2-Hydroperoxy-1-propanol und 1-Hydroperoxy-2-propanol erhalten wird.

Damit ist außer der Reaktion von Propylenoxid mit Wasser eine zweite Bildungsmöglichkeit für Propylenglykol als Nebenprodukt gegeben.

Für die weitere Umsetzung ist es nun außerordentlich vorteilhaft, dass das Rohpropylenoxid ohne weitere Reinigung in das erfindungsgemäße Verfahren eingesetzt werden kann.

Hierbei wird nun besagtes Rohpropylenoxid mit Wasser zu einem Gemisch der Propylenglykole umgesetzt. Es können vorzugsweise das beim Stand der Technik beschriebene Verfahren und die darin angegebenen Reaktionsbedingungen sowie Reaktoren für die Umsetzung verwendet werden. Weiterhin sind aber auch alle möglichen Verfahren zur katalytischen Addition von Wasser an Propylenoxid anwendbar, beispielsweise die in der WO 99/31034 beschriebene Methode.

Vorzugsweise kann das Rohpropylenoxid mit einem stöchiometrischen Überschuss an Wasser bei einer Temperatur von 180 bis 220°C und einem Druck zwischen 15 und 25 bar umgesetzt werden, etwa in einem Rohrreaktor oder in hintereinander geschalteten Rohrreaktoren. Die entstehenden Propylenglykole liegen dann in Form eines Gemisches vor, bestehend im Wesentlichen aus Propylenglykol, dem Di- und Tripropylenglykol und Polypropylenglykolen.

Demzufolge ist das erfindungsgemäße Verfahren dann dadurch gekennzeichnet, dass in Stufe (ii) Propylenoxid mit Wasser bei einer Temperatur von 180 bis 220°C und einem Druck zwischen 15 und 25 bar umgesetzt wird.

Die Verweilzeiten im Reaktor bzw. in den Reaktoren richten sich im Wesentlichen nach den gewünschten Umsätzen. Im Allgemeinen liegen sie bei weniger als 5 Stunden, bevorzugt bei weniger als 3 Stunden, weiter bevorzugt bei weniger als 1 Stunde und besonders bevorzugt bei etwa einer halben Stunde.

Vorzugsweise wird das in Stufe (ii) erhaltene Gemisch in eine Entwässerungsapparatur überführt, in der in einer mehrstufigen Verdampfung das überschüssige Wasser abgetrennt wird. Dieses kann in die Reaktion mit dem Propylenoxid zurückgeführt werden. Als Apparaturen für die Entwässerung können herkömmliche Destillationskolonnen verwendet werden, in denen das Wasser als leichtsiedende Fraktion über Kopf abdestilliert wird. Mit dem Sumpf oder über den Seitenabzug solcher Apparaturen kann das Glykolgemisch isoliert werden.

Eine bevorzugte Ausführungsform des erfindungsgemäßen Verfahrens ist demzufolge dadurch gekennzeichnet, dass dem in Stufe (ii) erhaltenen Gemisch vor der Abtrennung der Propylenglykole in Stufe (iii) das Wasser entzogen wird.

Das in der Stufe der Entwässerung erhaltene Gemisch weist vor der Überführung in die zur Abtrennung der Propylenglykole vorgesehene Apparatur vorzugsweise noch einen Wassergehalt von unter 5 Gew.-% auf.

In Stufe (iii) werden nun die in Stufe (i) und Stufe (ii) erhaltenen Glykole abgetrennt. Dabei wird das in Stufe (i) als Nebenausbeute erhaltene und abgetrennte Glykolgemisch, wie vorstehend beschrieben, mit dem aus der Stufe der Entwässerung erhaltenen Glykolgemisch vereinigt und zur Abtrennung der einzelnen Glykole gemeinsam destilliert.

Vorzugsweise werden dabei vor der Zusammenführung der Glykolgemische aus Stufe (i) und Stufe (ii) die im Gemisch der Stufe (i) enthaltenen Methoxypropanole sowie das Lösungsmittel, nämlich Methanol, abgetrennt.

Erfindungsgemäß wird nun ein Eduktstrom, der das Glykolgemisch enthält, einer Trennwandkolonne kontinuierlich zugeführt. Erfindungsgemäß wird dabei eine Trennwandkolonne mit zwei Seitenabzügen verwendet, die übereinander angeordnet sind, und eine damit thermisch gekoppelte Kolonne, die aus dem unteren Bereich der Trennwandkolonne gespeist wird. Bei der thermisch gekoppelten Kolonne handelt es sich dabei um eine herkömmliche Verstärkungskolonne, die ohne Verdampfer betrieben wird. Die benötigte Wärmeleistung dieser Kolonne wird über den ausgetauschten Dampfstrom eingetragen.

Trennwandkolonnen sind aus dem Stand der Technik bekannt. Sie stellen eine Weiterentwicklung von Destillationskolonnen dar, die nur über einen Seitenabzug, jedoch keine Trennwand verfügen. Die Anwendungsmöglichkeit des zuletzt genannten Kolonnentyps ist eingeschränkt, weil die an den Seitenabzugsstellen entnommenen Produkte nie völlig rein sind. Bei Seitenabnahmen im Verstärkungsteil der Kolonne, die üblicherweise in flüssiger Form erfolgen, enthält das Seitenprodukt noch Anteile an leichtsiedenden Komponenten, die über Kopf abgetrennt werden sollen. Bei Seitenabnahmen im Abtriebsteil der Kolonne, die meist dampfförmig erfolgen, weist das Seitenprodukt noch Hochsiederanteile auf. Die Verwendung von konventionellen Seitenabzugskolonnen ist daher auf Fälle begrenzt, in denen verunreinigte Seitenprodukte zulässig sind.

Beim Einbau einer Trennwand in eine solche Kolonne kann die Trennwirkung verbessert werden. Bei dieser Bauart ist es möglich, Seitenprodukte in reiner Form zu entnehmen. Im mittleren Bereich oberhalb und unterhalb der Zulaufstelle und der Seitenentnahmestellen ist eine Trennwand angebracht, wobei diese fest verschweißt oder auch nur gesteckt werden kann. Sie dichtet den Entnahmeteil gegenüber dem Zulaufteil ab und unterbindet in diesem Kolonnenteil eine Quervermischung von Flüssigkeits- und Brüdenströmen über den gesamten Kolonnenquerschnitt. Hierdurch verringert sich bei der Auftrennung von Vielstoffgemischen, deren Komponenten ähnliche Siedepunkte besitzen, die Zahl der insgesamt benötigten Destillationskolonnen.

Dieser Kolonnentyp wurde beispielsweise zur Trennung einer Komponentenvorlage aus Methan, Ethan, Propan und Butan verwendet (US 2,471,134), zur Trennung eines Gemisches von Benzol, Toluol und Xylol (US 4,230,533), zur Trennung eines Gemisches aus n-Hexan, n-Heptan und n-Octan (EP 0 122 367), zur Trennung azeotrop siedender Mischungen (EP 0 133 510) sowie zur Durchführung chemischer Reaktionen unter gleichzeitiger Destillation der Produkte (EP 0 126 288).

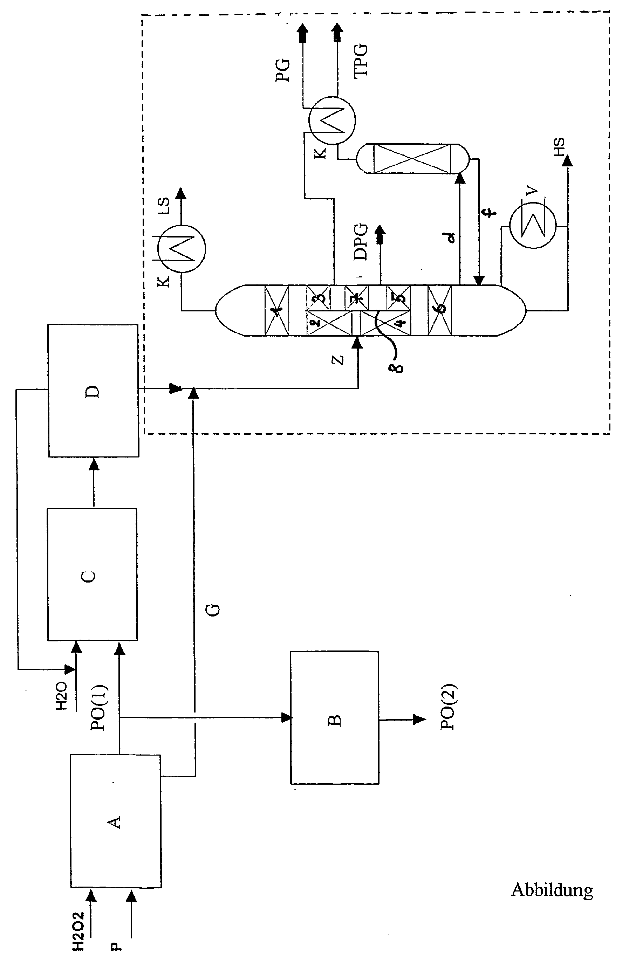

Eine solche Trennwandkolonne, wie sie schematisch in der Abbildung dargestellt ist, besitzt beispielsweise vorzugsweise 15 bis 60, besonders bevorzugt 20 bis 40, theoretische Trennstufen. Mit dieser Ausführung kann das erfindungsgemäße Verfahren besonders günstig durchgeführt werden.

Dabei weist der obere gemeinsame Teilbereich des Zulauf- und des Entnahmeteils 1 der Kolonne vorzugsweise 5 bis 50 %, besonders bevorzugt 15 bis 30 %, der Verstärkungsteil 2 des Zulaufteils vorzugsweise 5 bis 50 %, besonders bevorzugt 15 bis 30 %, der Abtriebsteil 4 des Zulaufteils vorzugsweise 5 bis 50 %, besonders bevorzugt 15 bis 30 %, der Abtriebsteil 3 des Entnahmeteils vorzugsweise 5 bis 50 %, besonders bevorzugt 15 bis 30 %, der Verstärkungsteil 5 des Entnahmeteils vorzugsweise 5 bis 50 %, besonders bevorzugt 15 bis 30 %, der gemeinsame untere Teilbereich 6 des Zulauf- und des Entnahmeteils der Kolonne vorzugsweise 5 bis 50 %, besonders bevorzugt 15 bis 30 % und der Bereich der thermischen Kopplung 7 der beiden Seitenabzüge vorzugsweise 5 bis 50 %, besonders bevorzugt 15 bis 30 % der Gesamtzahl der theoretischen Trennstufen der Kolonne auf.

Vorzugsweise beträgt die Summe der Zahl der theoretischen Trennstufen der Teilbereiche 2 und 4 im Zulaufteil 80 bis 110 %, weiter bevorzugt 90 bis 100 %, der Summe der Zahl der Trennstufen der Teilbereiche 3, 5 und 7 im Entnahmeteil.

Gleichfalls günstig ist es, die Zulaufstelle Z und die Seitenabzugsstellen, aus denen die Glykole PG und DPG entnommen werden, hinsichtlich der Lage der theoretischen Trennstufen auf unterschiedlicher Höhe in der Kolonne anzuordnen. Vorzugsweise ist die Zulaufstelle Z um 1 bis 8, besonders bevorzugt um 3 bis 5, theoretische Trennstufen höher oder niedriger angeordnet als die Seitenabzugsstellen.

Die beim erfindungsgemäßen Verfahren verwendete Trennwandkolonne kann vorzugsweise sowohl als Packungskolonne mit Füllkörpern oder geordneten Packungen oder als Bodenkolonne ausgeführt werden. Beispielsweise können als geordnete Packungen Blech- oder Gewebepackungen mit einer spezifischen Oberfläche von 100 bis 1000 m²/m³, bevorzugt etwa 250 bis 750 m²/m³ eingesetzt werden. Solche Packungen bieten eine hohe Trennleistung bei gleichzeitig niedrigem Druckverlust pro Trennstufe.

Vorzugsweise werden bei vorstehend genannter Ausführung der Kolonne der durch die Trennwand 8 unterteilte Teilbereich der Kolonne, bestehend aus dem Verstärkungsteil 2 des Zulaufteils, dem Abtriebsteil 3 des Entnahmeteils, dem Abtriebsteil 4 des Zulaufteils und dem Verstärkungsteil 5 des Entnahmeteils oder Teilen davon, mit geordneten Packungen oder Füllkörpern bestückt und die Trennwand in diesen Teilbereichen wärmeisolierend ausgeführt.

Zweckmäßigerweise wird der Produktstrom mittels einer Pumpe oder über eine statische Zulaufhöhe von mindestens 1 m mengengeregelt in den Zulaufteil aufgegeben. Vorzugsweise erfolgt diese Zugabe über eine Kaskadenregelung in Verbindung mit der Flüssigkeitsstandregelung des Auffangraumes des Zulaufteils. Dabei wird die Regelung so eingestellt, dass die auf das Verstärkungsteil des Zulaufteils aufgegebene Flüssigkeitsmenge nicht unter 30 % des Normalwertes sinken kann. Es hat sich gezeigt, dass eine derartige Vorgehensweise zur Kompensation von störenden Schwankungen bezüglich der Zulaufmenge oder der Zulaufkonzentration wichtig ist.

Ähnlich wichtig ist, dass die Aufteilung der aus dem Abtriebsteil des Entnahmeteils der Kolonne ablaufenden Flüssigkeit auf den Seitenabzug und auf den Verstärkungsteil des Entnahmeteils durch eine Regelung so eingestellt wird, dass die auf den Teilbereich aufgegebene Flüssigkeitsmenge nicht unter 30 % des Normalwertes sinken kann.

Die Einhaltung dieser Voraussetzungen muss durch entsprechende Regelvorschriften sichergestellt werden.

Regelungsmechanismen zum Betreiben von Trennwandkolonnen wurden beispielsweise beschrieben in Chem. Eng. Technol. 10 (1987) 92-98, Chem.-Ing.-Technol. 61 (1989) Nr. 1, 16-25, Gas Separation and Purification 4 (1990) 109-114, Process Engineering 2 (1993) 33-34, Trans IChemE 72 (1994) Part A 639-644, Chemical Engineering 7 (1997) 72-76. Die bei diesem Stand der Technik angegebenen Regelungsmechanismen können auch für das erfindungsgemäße Verfahren angewendet bzw. auf dieses übertragen werden.

Für die kontinuierlich betriebene Abtrennung der Glykole hat sich nachfolgend beschriebenes Regelungsprinzip als besonders günstig erwiesen. Es ist in der Lage, Lastschwankungen gut zu verkraften. Vorzugsweise erfolgt somit die Destillatentnahme temperaturgeregelt.

Im oberen Kolonnenteil 1 ist eine Temperaturregelung vorgesehen, die als Stellgröße die Ablaufmenge, das Rücklaufverhältnis oder bevorzugt die Rücklaufmenge nutzt. Dabei befindet sich die Messstelle für die Temperaturregelung vorzugsweise um 3 bis 8, weiter bevorzugt um 4 bis 6, theoretische Trennstufen unterhalb des oberen Endes der Kolonne.

Durch eine geeignete Temperatureinstellung wird dann die aus dem oberen Kolonnenteil 1 ablaufende Flüssigkeit am oberen Ende der Trennwand 8 so aufgeteilt, dass das Verhältnis des Flüssigkeitsstroms zum Zulaufteil zu dem zum Entnahmeteil vorzugsweise 0,1 bis 1,0, mehr bevorzugt 0,3 bis 0,6, beträgt.

Vorzugsweise wird bei dieser Methode die ablaufende Flüssigkeit in einem in oder außerhalb der Kolonne angeordneten Auffangraum gesammelt, woraus sie dann kontinuierlich in die Kolonne eingespeist wird. Dieser Auffangraum kann somit die Aufgabe einer Pumpenvorlage übernehmen oder für eine ausreichend hohe statische Flüssigkeitshöhe sorgen, die eine durch Stellorgane, beispielsweise Ventile, geregelte Flüssigkeitsweiterleitung ermöglicht. Bei der Verwendung von gepackten Kolonnen wird die Flüssigkeit zunächst in Sammler gefasst und von dort aus in einen innenliegenden oder außenliegenden Auffangraum geleitet.

Der Brüdenstrom am unteren Ende der Trennwand 8 wird durch die Wahl und/oder Dimensionierung der Trenneinbauten und/oder den Einbau druckmindernder Vorrichtungen, beispielsweise von Blenden, so eingestellt, dass das Verhältnis des Brüdenstroms im Zulaufteil zu dem des Entnahmeteils vorzugsweise 0,8 bis 1,2, bevorzugt 0,9 bis 1,1, beträgt.

Beim vorstehend genannten Regelprinzip ist des weiteren eine Temperaturregelung im unteren gemeinsamen Kolonnenteil 6 vorgesehen, die als Stellgröße die Sumpfentnahmemenge nutzt. Somit kann die Entnahme des Sumpfprodukts temperaturgeregelt erfolgen. Dabei ist die Messstelle für die Temperaturregelung vorzugsweise um 3 bis 6, besonders bevorzugt 4 bis 6, theoretische Trennstufen oberhalb des unteren Endes der Kolonne angeordnet.

Zusätzlich kann für die genannte Regelung für die untere Seitenentnahmemenge die Standregelung am Kolonnensumpf als Stellgröße und für die obere Seitenentnahmemenge eine Temperaturregelung im geteilten Kolonnenbereich vorgesehen werden.

Als Stellgröße der Heizleistung kann auch der Differenzdruck über die Kolonne genutzt werden. Günstigerweise wird die Destillation bei einem Kopfdruck zwischen 5 und 500 mbar, bevorzugt zwischen 10 und 200 mbar, durchgeführt. Dementsprechend wird zur Einhaltung dieses Druckbereiches die Heizleistung des Verdampfers am Kolonnenboden gewählt.

Unter diesen Druckbedingungen liegt dann die Destillationstemperatur zwischen 50 und 200°C, bevorzugt zwischen 80 und 180°C, gemessen an den Seitenabzügen.

Um die Trennwandkolonne störungsfrei betreiben zu können, werden die genannten Regelmechanismen zumeist in Kombination angewendet.

Vorzugsweise beträgt die Summe der Zahl der theoretischen Trennstufen des Verstärkungs- und Abtriebsteils 2 und 4 im Zulaufteil 80 bis 110 %, besonders bevorzugt 90 bis 100 %, der Summe der Zahl der Trennstufen der Teilbereiche des Verstärkungs- und Abtriebsteils 3 und 5 im Entnahmeteil sowie des Bereichs der thermischen Kopplung 7 der Seitenabzugsstellen.

Die Aufteilung der aus dem Abtriebsteil 3 des Entnahmeteils der Kolonne ablaufenden Flüssigkeit auf den Seitenabzug und auf den Verstärkungsteil 5 des Entnahmeteils wird dabei durch eine Regelung so eingestellt, dass die auf den Teilbereich aufgegebene Flüssigkeitsmenge nicht unter 30 % des Normalwertes sinken kann.

Beispielsweise kann mit dem beschriebenem Regelprinzip der die Propylenglykole enthaltende Produktstrom so aufgetrennt werden, dass über den Kopf der Trennwandkolonne die Leichtsieder LS, die z. B. Wasser und Lösungsmittel enthalten, durch Kondensation mit dem Kondensator K entnommen werden können. Die Mittelsieder werden über die obere Seitenentnahmestelle in Form des Propylenglykols PG und über die untere Seitenentnahmestelle in Form des Dipropylenglykols DPG abgezogen. Die Hochsieder HS, die das Tripropylenglykol TPG enthalten, können mit dem Sumpf der Kolonne erhalten werden.

Bei der Trennung von Mehrstoffgemischen in eine Leichtsieder-, Mittelsiederfraktion- und Hochsiederfraktion existieren üblicherweise Spezifikationen über den maximal zulässigen Anteil an Leichtsiedern und Hochsiedern in der Mittelsiederfraktion. Hierbei werden entweder einzelne für das Trennproblem kritische Komponenten, sogenannte Schlüsselkomponenten, oder die Summe von mehreren Schlüsselkomponenten spezifiziert.

Die Einhaltung der Spezifikation für die Hochsieder in der Mittelsiederfraktion wird vorzugsweise über das Aufteilungsverhältnis der Flüssigkeitsmenge am oberen Ende der Trennwand 8 geregelt. Dabei wird das Aufteilungsverhältnis so eingestellt, dass die Konzentration an Schlüsselkomponenten für die Hochsiederfraktion in der Flüssigkeit am oberen Ende der Trennwand 10 bis 80 Gew.-%, bevorzugt 30 bis 50 Gew.-%, des Wertes ausmacht, der im Seitenabzug erzielt werden soll. Die Flüssigkeitsaufteilung kann dann so eingestellt werden, dass bei höheren Gehalten an Schlüsselkomponenten der Hochsiederfraktion mehr und bei niedrigeren Gehalten an Schlüsselkomponenten weniger Flüssigkeit auf den Zulaufteil geleitet wird.

Entsprechend wird die Spezifikation für die Leichtsieder LS in der Mittelsiederfraktion durch die Heizleistung geregelt. Hierbei wird die Heizleistung im Verdampfer V so eingestellt, dass die Konzentration an Schlüsselkomponenten für die Leichtsiederfraktion in der Flüssigkeit am unteren Ende der Trennwand 10 bis 80 Gew.-%, bevorzugt 30 bis 50 Gew.-%, des Wertes ausmacht, der im Seitenabzugsprodukt erzielt werden soll. Somit wird die Heizleistung dahingehend eingestellt, dass bei höherem Gehalt an Schlüsselkomponenten der Leichtsiederfraktion die Heizleistung erhöht und bei niedrigerem Gehalt an Schlüsselkomponenten der Leichtsiederfraktion die Heizleistung verringert wird.

Die Konzentration von Leicht- und Hochsiedern in der Mittelsiederfraktion kann nach üblichen Analysemethoden ermittelt werden. Beispielsweise kann zur Detektion Infrarot-Spektroskopie verwendet werden, wobei die im Reaktionsgemisch vorliegenden Verbindungen an Hand ihrer charakteristischen Absorptionen identifiziert werden. Diese Messungen können inline direkt in der Kolonne vorgenommen werden. Bevorzugt werden jedoch gaschromatographische Methoden verwendet. Hierbei ist dann vorgesehen, dass das obere und untere Ende der Trennwand Probeentnahmemöglichkeiten aufweisen. Somit können aus der Kolonne kontinuierlich oder in zeitlichen Abständen flüssige oder gasförmige Proben entnommen und hinsichtlich ihrer Zusammensetzung untersucht werden. In Abhängigkeit von der Zusammensetzung kann dann auf die entsprechenden Regelmechanismen zurückgegriffen werden.

Es ist ein Ziel des erfindungsgemäßen Verfahrens, die Mittelsieder Propylenglykol PG und Dipropylenglykol DPG mit einer Reinheit von jeweils vorzugsweise größer 95 % und weiter bevorzugt größer 99 % zur Verfügung zu stellen.

Aus dem Sumpf der Trennwandkolonne wird nun ein das Tripropylenglykol TPG enthaltende Gemisch in eine Destillationskolonne überführt, die mit der Trennwandkolonne thermisch gekoppelt ist.

Erfindungsgemäß erfolgt die Energiezufuhr über die Trennwandkolonne mit dem Verdampfer V. Der Dampf- und Flüssigkeitsaustausch d und f erfolgt im unteren Bereich der Trennwandkolonne sowie der damit thermisch gekoppelten Kolonne.

In der thermisch gekoppelten Kolonne kann bei einem Druck von vorzugsweise 5 bis 500 mbar, besonders bevorzugt 10 bis 200 mbar, und einer Temperatur von vorzugsweise 100 bis 200°C, besonders bevorzugt 120 bis 180°C, das Tripropylenglykol TPG über den Kopf der Kolonne abdestilliert werden. Es wird dann über den Kondensator K kondensiert, wobei der Strom aus Dipropylenglykol DPG als Kühlmittel verwendet werden kann. Vorzugsweise wird das Tripropylenglykol in einer Reinheit von mindestens 95 % erhalten.

Kondensierende Produkte mit höherem Siedepunkt als dem Siedepunkt von Tripropylenglykol TPG werden mit dem Sumpf der thermisch gekoppelten Kolonne als Flüssigkeitsstrom f in die Trennwandkolonne zurückgeführt. Dort können sie letztendlich als Hochsieder HS mit dem Sumpf der Kolonne entnommen und beispielsweise einer Verbrennung zugeführt werden. Solche Sumpfprodukte sind beispielsweise Polypropylenglykole.

Auch die thermisch gekoppelte Kolonne, in der das Tripropylenglykol abgetrennt wird, kann als Packungskolonne mit Füllkörpern oder geordneten Packungen oder als Bodenkolonne ausgeführt werden. Beispielsweise können als geordnete Packungen Blech- oder Gewebepackungen mit einer spezifischen Oberfläche von 100 bis 1000 m²/m³, bevorzugt etwa 250 bis 750 m²/m³ eingesetzt werden. Solche Packungen bieten eine hohe Trennleistung bei gleichzeitig niedrigem Druckverlust pro Trennstufe.

Besonders die Kopplung des kontinuierlichen Herstellprozesses für Propylenoxid mit der Herstellung der Propylenglykole, die gleichfalls kontinuierlich durchgeführt wird, macht das erfindungsgemäß kontinuierlich durchgeführte Verfahren für die industrielle Anwendung außerordentlich vorteilhaft. Unter kontinuierlich wird somit verstanden, dass die Stufen (i), (ii) und (iii) des erfindungsgemäßen Verfahrens gleichzeitig durchgeführt werden.

Für die kontinuierliche Herstellung von Propylenglykol, Di- und Tripropylenglykol ergibt sich somit ein rückwärtsintegriertes Verfahren bis zum Propylen. Da auch die Reindestillation des Propylenoxids sowie die beim Stand der Technik beschriebene Destillation in drei Kolonnen entfällt, können bei gleichzeitig reduziertem apparativem Aufwand vergleichsweise ca. 30 % an Energiekosten eingespart werden.

Das erfindungsgemäße Verfahren soll nun nochmals anhand der Abbildung im Überblick schematisch dargestellt werden.

Im Bereich A erfolgt die Umsetzung von Wasserstoffperoxid mit Propylen P zu Propylenoxid. Dabei werden nach der Aufarbeitung rohes Propylenoxid PO(1) und als Nebenausbeute die Propylenglykole G erhalten.

Das rohe Propylenoxid PO(1) wird nun in den Bereich C überführt, wo es mit Wasser zu Propylenglykolen umgesetzt wird. Im Bereich D wird diesen das Wasser entzogen, das in die Umsetzung mit Propylenoxid PO(1) zu den Propylenglykolen zurückgeführt werden kann.

Der aus dem Bereich D erhaltene Eduktstrom wird mit der Nebenausbeute an Propylenglykolen G vereinigt und über die Zulaufstelle Z in die Trennwandkolonne eingespeist. In der Trennwandkolonne werden Propylenglykol PG und Dipropylenglykol DPG und in der damit thermisch gekoppelten Kolonne Tripropylenglykol TPG abgetrennt.

Ferner ist es auch möglich, aus dem Produktstrom PO(1) einen Teilstrom abzuzweigen, der im Bereich B zu reinem Propylenoxid PO(2) verarbeitet werden kann, das beispielsweise in einer Reinheit von mehr als 99,9 % an Wertstoff vorliegen kann.

Für das erfindungsgemäße Verfahren kann Propylen der Qualitätsstufe "chemical grade" eingesetzt werden. Ein solches Propylen enthält Propan, wobei Propylen und Propan im Volumenverhältnis von ca. 97:3 bis 95:5 vorliegen.

Das zur Umsetzung eingesetzte Wasserstoffperoxid liegt vorzugsweise als wässerige Wasserstoffperoxidlösung vor. Es kann beispielsweise über das Anthrachinonverfahren, wie es in "Ullmann's Encyclopedia of Industrial Chemistry", 5. Auflage, Band 13, Seiten 447 bis 456, beschrieben ist, hergestellt werden.

Ebenso ist es denkbar, zur Wasserstoffperoxidgewinnung Schwefelsäure durch anodische Oxidation unter gleichzeitiger kathodischer Wasserstoffentwicklung in Peroxodischwefelsäure zu überführen. Die Hydrolyse der Peroxodischwefelsäure führt dann auf dem Weg über Peroxoschwefelsäure zu Wasserstoffperoxid und Schwefelsäure, die damit zurückgewonnen wird.

Möglich ist selbstverständlich auch die Darstellung von Wasserstoffperoxid aus den Elementen.

Als Lösungsmittel für die Propylenoxidherstellung können vorzugsweise alle Lösungsmittel verwendet werden, die die in die Oxiransynthese eingesetzten Edukte ganz oder wenigstens teilweise lösen. Beispielsweise können verwendet werden Wasser; Alkohole, bevorzugt niedere Alkohole, weiter bevorzugt Alkohole mit weniger als 6 Kohlenstoffatomen wie beispielsweise Methanol, Ethanol, Propanole, Butanole, Pentanole, Diole oder Polyole, bevorzugt solche mit weniger als 6 Kohlenstoffatomen; Ether, wie beispielsweise Diethylether, Tetrahydrofuran, Dioxan, 1,2-Diethoxyethan, 2-Methoxyethanol; Ester, wie beispielsweise Methylacetat oder Butyrolacton; Amide, wie beispielsweise Dimethylformamid, Dimethylacetamid, N-Methylpyrrolidon; Ketone, wie beispielsweise Aceton; Nitrile, wie beispielsweise Acetonitril; Sulfoxide, wie beispielsweise Dimethylsulfoxid; aliphatische, cycloaliphatische und aromatische Kohlenwasserstoffe, oder Gemische aus zwei oder mehr der vorgenannten Verbindungen.

Bevorzugt werden Alkohole eingesetzt. Dabei ist der Einsatz von Methanol als Lösungsmittel besonders bevorzugt.

Als Katalysatoren für die Propylenoxidherstellung werden bevorzugt solche verwendet, die ein poröses oxidisches Material, wie z. B. einen Zeolith, umfassen. Vorzugsweise werden Katalysatoren eingesetzt, die als poröses oxidisches Material einen Titan-, Germanium-, Tellur-, Vanadium-, Chrom-, Niob- oder Zirkoniumhaltigen Zeolith umfassen.

Dabei sind im einzelnen Titan-, Germanium-, Tellur-, Vanadium-, Chrom-, Niob-, Zirkonium-haltige Zeolithe mit Pentasil-Zeolith-Struktur, insbesondere die Typen mit röntgenografischer Zuordnung zur ABW-, ACO-, AEI-, AEL-, AEN-, AET-, AFG-, AFI-, AFN-, AFO-, AFR-, AFS-, AFT-, AFX-, AFY-, AHT-, ANA-, APC-, APD-, AST-, ATN-, ATO-, ATS-, ATT-, ATV-, AWO-, AWW-, BEA-, BIK-, BOG-, BPH-, BRE-, CAN-, CAS-, CFI-, CGF-, CGS-, CHA-, CHI-, CLO-, CON-, CZP-, DAC-, DDR-, DFO-, DFT-, DOH-, DON-, EAB-, EDI-, EMT-, EPI-, ERI-, ESV-, EUO-, FAU-, FER-, GIS-, GME-, GOO-, HEU-, IFR-, ISV-, ITE-, JBW-, KFI-, LAU-, LEV-, LIO-, LOS-, LOV-, LTA-, LTL-, LTN-, MAZ-, MEI-, MEL-, MEP-, MER-, MFI-, MFS-, MON-, MOR-, MSO-, MTF-, MTN-, MTT-, MTW-, MWW-, NAT-, NES-, NON-, OFF-, OSI-, PAR-, PAU-, PHI-, RHO-, RON-, RSN-, RTE-, RTH-, RUT-, SAO-, SAT-, SBE-, SBS-, SBT-, SFF-, SGT-, SOD-, STF-, STI-, STT-, TER-, THO-, TON-, TSC-, VET-, VFI-, VNI-, VSV-, WIE-, WEN-, YUG-, ZON-Struktur sowie zu Mischstrukturen aus zwei oder mehr der vorgenannten Strukturen zu nennen. Denkbar sind für den Einsatz im erfindungsgemäßen Verfahren weiterhin titanhaltige Zeolithe mit der Struktur des ITQ-4, SSZ-24, TTM-1, UTD-1, CIT-1 oder CIT-5. Als weitere titanhaltige Zeolithe sind solche mit der Struktur des ZSM-48 oder ZSM-12 zu nennen.

Als besonders bevorzugt sind Ti-Zeolithe mit MFI-, MEL- oder MFI/MEL-Mischstruktur anzusehen. Ganz besonders bevorzugt sind im Einzelnen die Titanenthaltenden Zeolith-Katalysatoren, die im allgemeinen als "TS-1", "TS-2", "TS-3" bezeichnet werden, sowie Ti-Zeolithe mit einer zu β-Zeolith isomorphen Gerüststruktur.

Sehr günstig ist die Verwendung eines heterogenen Katalysator, der das Titan-haltige Silikalit TS-1 umfasst.

Im Allgemeinen liegt die Reaktionstemperatur für die Herstellung des Propylenoxids in den Stufen (α) und (γ) im Bereich von 0 bis 120°C, bevorzugt im Bereich von 10 bis 100°C und weiter bevorzugt im Bereich von 20 bis 90°C. Die auftretenden Drücke reichen dabei von 1 bis 100 bar, vorzugsweise 1 bis 40 bar, besonders bevorzugt 1 bis 30 bar. Bevorzugt wird bei Drücken gearbeitet, unter denen keine Gasphase vorliegt.

Die Konzentration von Propylen und Wasserstoffperoxid im Eduktstrom wird im Allgemeinen so gewählt, dass das molare Verhältnis bevorzugt im Bereich von 0,7 bis 20, weiter bevorzugt im Bereich von 0,8 bis 5,0, besonders bevorzugt im Bereich von 0,9 bis 2,0 und insbesondere im Bereich von 1,0 bis 1,6 liegt.

Bei der Propylenoxidherstellung richten sich die Verweilzeiten im Reaktor bzw. in den Reaktoren dabei im Wesentlichen nach den gewünschten Umsätzen. Im Allgemeinen liegen sie bei weniger als 5 Stunden, bevorzugt bei weniger als 3 Stunden, weiter bevorzugt bei weniger als 1 Stunde und besonders bevorzugt bei etwa einer halben Stunde.

Zur Reduktion der bei der Propylenoxidherstellung im Produktgemisch enthaltenen Hydroperoxyalkohole können die in der DE 101 05 527.7 beschriebenen Methoden herangezogen werden.

Beispielsweise ist es möglich, Phosphor(III)-Verbindungen, beispielsweise Phosphortrichlorid, Phosphane (z.B. Triphenylphosphin, Tributylphosphin), Phosphorige Säure bzw. deren Salze oder Natriumhypophosphit, einzusetzen.

Auch die reduktive Umsetzung mit Schwefel(II)-Verbindungen wie beispielsweise Schwefelwasserstoff oder Salze davon, Natriumpolysulfide, Dimethylsulfid, Tetrahydrothiophen, Bis-(hydroxyethyl)-sulfid oder Natriumthiosulfat sowie mit Schwefel(IV)-Verbindungen, wie beispielsweise Schweflige Säure und deren Salze, Natriumbisulfit oder Thioharnstoff-S-oxid, ist möglich.

Weitere Reduktionsmittel sind Nitrite, beispielsweise Natriumnitrit oder Isoamylnitrit. Auch α-Hydroxycarbonylverbindungen wie Hydroxyaceton, Dihydroxyaceton, 2-Hydroxycyclopentanon (Glutaroin), 2-Hydroxycyclohexanon (Adipoin), Glucose sowie andere reduzierbare Zucker sind geeignet. Endiole wie Ascorbinsäure oder Verbindungen, welche eine Bor-Wasserstoff-Bindung enthalten, beispielsweise Natriumborhydrid oder Natriumcyanborhydrid, sind gleichfalls verwendbar.

Bevorzugt werden zur Reduktion von α-Hydroperoxyalkohol-haltigen Produktgemischen jedoch katalytische Hydrierverfahren mit Wasserstoff gewählt, die in homogener wie auch heterogener Phase durchgeführt werden können. Der Hydrierkatalysator weist dabei mindestens ein Aktivmetall aus der VIIb-, der VIII-, der Ia- sowie der Ib-Nebengruppe des Periodensystems der Elemente, einzeln oder als Gemisch aus zwei oder mehr davon, auf. Beispielsweise können Palladium (Pd), Platin (Pt), Rhodium (Rh), Ruthenium (Ru), Iridium (Ir), Osmium (Os), Eisen (Fe), Cobalt (Co), Nickel (Ni) sowie Kupfer (Cu), bevorzugt Pd, Pt, Rh, Ru und Ir, besonders bevorzugt Pd eingesetzt werden. Diese Katalysatoren sind sowohl in Pulverform einsetzbar wie auch als Aktivmetallkörper. Dabei kommen bevorzugt Folien, Drähte, Netze, Granalien und Kristallitpulver, hergestellt aus mindestens einem Aktivmetall oder einer Mischung aus zwei oder mehr davon, zur Anwendung. Weiterhin sind auch Aktivmetalloxide, beispielsweise als Suspensionen mindestens eines Aktivmetalls oder einer Mischung aus zwei oder mehr davon, einsetzbar.

Weiterer Gegenstand der Erfindung ist ferner eine Vorrichtung zur Durchführung eines kontinuierlichen Verfahrens zur Herstellung von Propylenglykolen, umfassend wenigstens einen Reaktor zur Herstellung von Propylenoxid, wenigstens einen Reaktor zur Umsetzung des Propylenoxids mit Wasser zu Propylenglykolen, wenigstens eine Entwässerungsapparatur zur Entwässerung der wasserhaltigen Propylenglykole und wenigstens eine Trennwandkolonne mit zwei Seitenabzügen zur Abtrennung von Propylenglykol und Dipropylenglykol sowie eine damit thermisch gekoppelte Kolonne zur Abtrennung des Tripropylenglykols.

Eine besonders bevorzugte Ausführungsform der erfindungsgemäßen Vorrichtung ist dadurch gekennzeichnet, dass der wenigstens eine Reaktor aus einem isothermen Festbettreaktor zur Durchführung der Stufe (α), einem adiabatischen Festbettreaktor zur Durchführung der Stufe (γ) und einer Abtrennapparatur zur Durchführung der Stufe (β) besteht.

Dabei werden im isothermen bzw. adiabatischen Reaktor die Umsetzungen von Propylen mit Wasserstoffperoxid in den Stufe (α) und (γ) durchgeführt. In der Abtrennapparatur wird in Stufe (β) nicht umgesetztes Wasserstoffperoxid aus Stufe (α) abgetrennt und in Stufe (γ) erneut mit Propylen umgesetzt.

### Bezugszeichenliste der Abbildung

- 1: Gemeinsamer Teilbereich des Zulauf- und Entnahmeteils der Trennwandkolonne
- 2: Verstärkungsteil des Zulaufteils
- 3: Abtriebsteil des Entnahmeteils
- 4: Abtriebsteil des Zulaufteils
- 5: Verstärkungsteil des Entnahmeteils
- 6: Gemeinsamer Teilbereich des Zulauf- und Entnahmeteils
- 7: Bereich thermischer Kopplung
- 8: Trennwand

- P: Propylen
- A: Bereich der Propylenoxidherstellung
- PO(1): Rohes Propylenoxid
- B: Bereich der Propylenoxidaufarbeitung
- PO(2): Gereinigtes Propylenoxid
- G: Nebenausbeute Propylenglykole
- C: Bereich der Umsetzung von Propylenoxid mit Wasser
- D: Bereich der Entwässerung der Propylenglykole
- Z: Zulaufstelle
- LS: Leichtsieder
- PG: Propylenglykol
- DPG: Dipropylenglykol
- TPG: Tipropylenglykol
- HS: Hochsieder
- K: Kondensator
- V: Verdampfer

- d: Dampf
- f: Flüssigkeit

Waagrechte und diagonale oder diagonal angedeutete Linien in den Kolonnen symbolisieren Packungen mit Füllkörpern oder geordnete Packungen, die in der Kolonne vorhanden sein können.

## Patentansprüche

1. Verfahren zur kontinuierlichen Herstellung von Propylenglykolen, **dadurch gekennzeichnet, dass** es die Stufen (i) bis (iii) umfasst:
(i) Umsetzung von Propylen mit Wasserstoffperoxid unter Erhalt eines Gemischs, enthaltend Propylenoxid und Mono-, Di- und Tripropylenglykol als Nebenprodukte, wobei aus diesem Gemisch in einer Destillationskolonne über Sumpf ein Gemisch, enthaltend Mono-, Di- und Tripropylenglykol, und über Kopf Rohpropylenoxid abgetrennt werden;
(ii) Umsetzung des in Stufe (i) erhaltenen Rohpropylenoxids mit Wasser unter Erhalt eines Gemischs, enthaltend Mono-, Di- und Tripropylenglykol;
(iii) Vereinigung der in den Stufen (i) und (ii) erhaltenen Propylenglykolgemische und destillative Abtrennung der einzelnen Propylenglykole,
wobei in (i) eine wässerige Wasserstoffperoxidlösung eingesetzt wird und
wobei dem in (ii) erhaltenen Gemisch vor der Vereinigung und Abtrennung in Stufe (iii) Wasser entzogen wird.

2. verfahren nach Anspruch 1, **dadurch gekennzeichnet, dass** in Stufe (i) die Umsetzung von Propylen mit Wasserstoffperoxid mindestens die Stufen (α) bis (γ) umfasst:
(α) Umsetzung von Propylen mit Wasserstoffperoxid unter Erhalt einer Mischung, umfassend Propylenoxid und nicht umgesetztes Wasserstoffperoxid.
(β) Abtrennung des nicht umgesetzten Wasserstoffperoxids aus der aus Stufe (α) resultierenden Mischung,
(γ) Umsetzung des abgetrennten Wasserstoffperoxids aus Stufe (β) mit Propylen.

3. Verfahren nach Anspruch 1 oder 2, **dadurch gekennzeichnet, dass** in Stufe (i) als Nebenprodukt Propylenglykol durch Reduktion von 2-Hydroperoxy-1-propanol und 1-Hydroperoxy-2-propanol erhalten wird.

4. Verfahren nach einem der Ansprüche 1 bis 3, **dadurch gekennzeichnet, dass** in Stufe (ii) Propylenoxid mit Wasser bei einer Temperatur von 180 bis 220 °C und einem Druck von 15 bis 2.5 bar umgesetzt wird.

5. Verfahren nach einem der Anspruche 1 bis 4, **dadurch gekennzeichnet, dass** in Stufe (iii) die destillative Abtrennung in einer Trennwandkolonne mit zwei Seitenabzügen und einer damit thermisch gekoppelten Kolonne erfolgt, wobei aus dem oberen Seitenabzug der Trennwandkolonne Monopropylenglykol, aus dem unteren Seitenabzug Dipropylenglykol und aus der damit thermisch gekoppelten Kolonne Tripropylenglykol erhalten wird.

6. Verfahren nach Anspruch 5, **dadurch gekennzeichnet, dass** die Destillation in der Trennwandkolonne bei einem Druck von 5 bis 500 mbar und einer Temperatur von 50 bis 200°C durchgeführt wird.

7. Verfahren nach Anspruch 5 oder 6, **dadurch gekennzeichnet, dass** die Destillation in der thermisch gekoppelten Kolonne bei einem Druck von 5 bis 500 mbar und einer Temperatur von 100 bis 200°C durchgeführt wird.

8. Vorrichtung zur Durchführung eines kontinuierlichen Verfahrens zur Herstellung von Propylenglykolen gemäß einem der Ansprüche 5 bis 7, **dadurch gekennzeichnet, dass** die Vorrichtung wenigstens einen Reaktor zur Herstellung von Propylenoxid, wenigstens einen Reaktor zur Umsetzung des Propylenoxids mit Wasser zu Propylenglykolen, wenigstens eine Entwässerungsapparatur zur Entwässerung der wasserhaltigen Propylenglykole und wenigstens eine Trennwandkolonne mit zwei Seitenabzügen zur Abtrennung von Monopropylenglykol und Dipropylenglykol sowie eine damit thermisch gekoppelte Kolonne zur Abtrennung des Tripropylenglykols umfasst.

9. Vorrichtung nach Anspruch 8, **dadurch gekennzeichnet, dass** der wenigstens eine Reaktor zur Herstellung von Propylenoxid aus einem isothermen Festbettreaktor zur Durchführung der Stufe (α), einem adiabatischen Festbettreaktor zur Durchführung der Stufe (γ) und einer Abtrennapparatur zur Durchführung der Stufe (β) wie in Anspruch 2 definiert, besteht.

## Claims

1. A process for the continuous preparation of propylene glycols, which comprises the steps (i) to (ii):
(i) reacting propylene with hydrogen peroxide to give a mixture comprising propylene oxide and monopropylene, dipropylene and tripropylene glycols as by-products, with a mixture comprising monopropylene, dipropylene and tripropylene glycols being separated off from this mixture at the bottom of a distillation column and crude propylene oxide being separated off at the top of the column;
(ii) reacting the crude propylene oxide obtained in step (i) with water to give a mixture comprising monopropylene, dipropylene and tripropylene glycols;
(iii) combining the propylene glycol mixtures obtained in steps (i) and (ii) and separating off the individual propylene glycols by distillation,
wherein an aqueous hydrogen peroxide solution is used in (i) and water is removed from the mixture obtained in (ii) before the combination and separation in step (ii).

2. The process according to claim 1, wherein the reaction of propylene with hydrogen peroxide in step (i) comprises at least the steps (α) to (γ):
(α) reacting propylene with hydrogen peroxide to give a mixture comprising propylene oxide and unreacted hydrogen peroxide,
(β) separating the unreacted hydrogen peroxide from the mixture resulting from step (α),
(γ) reacting the hydrogen peroxide which has been separated off in stage (β) with propylene.

3. The process according to claim 1 or 2, wherein propylene glycol is obtained as by-product in step (i) by reduction of 2-hydroperoxy-1-propanol and 1-hydroperoxy-2-propanol.

4. The process according to any of claims 1 to 3, wherein, in step (ii), propylene oxide is reacted with water at from 180 to 220°C and a pressure of from 15 to 25 bar.

5. The process according to any of claims 1 to 4, wherein the separation by distillation in step (iii) is carried out in a dividing wall column having two side offtakes and a column which is thermally coupled therewith, with monopropylene glycol being obtained from the upper side offtake of the dividing wall column, dipropylene glycol being obtained from the lower side offtake and tripropylene glycol being obtained from the column which is thermally coupled therewith.

6. The process according to claim 5, wherein the distillation in the dividing wall column is carried out at from 50 to 200°C and a pressure of from 5 to 500 mbar.

7. The process according to claim 5 or 6, wherein the distillation in the thermally coupled column is carried out at from 100 to 200°C and a pressure of from 5 to 500 mbar.

8. An apparatus for carrying out a continuous process for preparing propylene glycols according to any of claims 5 to 7, comprising at least one reactor for preparing propylene oxide, at least one reactor for reacting the propylene oxide with water to form propylene glycols, at least one dewatering apparatus for dewatering the water-containing propylene glycols and at least one dividing wall column having two side offtakes for separating off monopropylene glycol and dipropylene glycol and a column which is thermally coupled therewith for separating off the tripropylene glycol.

9. The apparatus according to claim 8, wherein the reactors for the preparation of propylene oxide comprise an isothermal fixed-bed reactor for carrying for carrying out the step (α), an adiabatic fixed-bed reactor for carrying out the step (γ) and a separation apparatus for carrying out the step (β) as defined in claim 2.

## Revendications

1. Procédé de préparation en mode continu de propylèneglycols, **caractérisé en ce qu'**il comprend les étapes (i) à (iii) :
(i) réaction de propylène avec du peroxyde d'hydrogène sous obtention d'un mélange, contenant l'oxyde de propylène et du mono-, di- et tripropylèneglycol comme produits secondaires, un mélange contenant du mono-, di- et tripropylèneglycol étant séparé de ce mélange dans une colonne de distillation par le fond, et de l'oxyde de propylène brut par la tête;
(ii) réaction de l'oxyde de propylène brut obtenu à l'étape (i) avec de l'eau sous obtention d'un mélange, contenant du mono-, di- et tripropylèneglycol;
(iii) réunification des mélanges de propylèneglycol obtenus aux étapes (i) et (ii) et séparation par distillation des différents propylèneglycols,
une solution aqueuse de peroxyde d'hydrogène étant utilisée en (i) et de l'eau étant soutirée du mélange obtenu en (ii) avant la réunification et la séparation à l'étape (iii).

2. Procédé selon la revendication 1, **caractérisé en ce qu'**à l'étape (i) la réaction du propylène avec du peroxyde d'hydrogène comprend au moins les étapes (α) à (γ):
(α) réaction du propylène avec du peroxyde d'hydrogène sous obtention d'un mélange, comprenant de l'oxyde de propylène et du peroxyde d'hydrogène non réagi;
(β) séparation du peroxyde d'hydrogène non réagi du mélange résultant de l'étape (α);
(γ) réaction du peroxyde d'hydrogène séparé de l'étape (β) avec du propylène.

3. Procédé selon la revendication 1 ou 2, **caractérisé en ce qu'**à l'étape (i), on obtient comme produit secondaire du propylèneglycol par réduction de 2-hydroperoxy-1-propanol et de 1-hydroperoxy-2-propanol.

4. Procédé selon l'une des revendications 1 à 3, **caractérisé en ce qu'**à l'étape (ii), l'oxyde de propylène est mis à réagir avec de l'eau à une température de 180 à 220°C et à une pression de 15 à 25 bar.

5. Procédé selon l'une des revendications 1 à 4, **caractérisé en ce qu'**à l'étape (iii), la séparation par distillation se fait dans une colonne sectionnée avec deux soutirages latéraux et une colonne thermiquement couplée à celle-ci, du monopropylèneglycol étant obtenu du soutirage latéral supérieur de la colonne sectionnée, du dipropylèneglycol étant obtenu du soutirage latéral inférieur et du tripopylèneglycol étant obtenu de la colonne thermiquement couplée à celle-ci.

6. Procédé selon la revendication 5, **caractérisé en ce que** la distillation se fait dans une colonne sectionnée à une pression de 5 à 500 mbar et à une température de 50 à 200°C.

7. Procédé selon la revendication 5 ou 6, **caractérisé en ce que** la distillation se fait dans une colonne thermiquement couplée à une pression de 5 à 500 mbar et à une température de 100 à 200°C.

8. Dispositif d'exécution d'un procédé en mode continu de préparation de propylèneglycols selon l'une des revendications 5 à 7, **caractérisé en ce que** le dispositif comporte au moins un réacteur de préparation d'oxyde de propylène, au moins un réacteur de réaction de l'oxyde de propylène avec de l'eau en propylèneglycols, au moins un appareil de déshydratation pour la déshydratation des propylèneglycols contenant de l'eau et au moins une colonne sectionnée avec deux soutirages latéraux pour la séparation du monopropylèneglycol et du dipropylèneglycol ainsi qu'une colonne thermiquement couplée à celle-à de séparation du tripropylèneglycol.

9. Dispositif selon la revendication 8, **caractérisé en ce que** ledit au moins un réacteur de préparation d'oxyde de propylène est constitué d'un réacteur à lit fixe isothermique pour l'exécution de l'étape (α), d'un réacteur à lit fixe adiabatique pour l'exécution de l'étape (γ) et d'un appareil de séparation pour l'exécution de l'étape (β), telles qu'elles sont définies à la revendication 2.
